# EUROPEAN PATENT APPLICATION

(11) **EP 4 481 400 A1**
(43) Date of publication of application: **25.12.2024**
(21) Application number: 23756461.2
(22) Date of filing: 16.02.2023
(51) Int. Cl.: G01N 35/10

(54) **MEASURING DEVICE**

(30) Priority: 17.02.2022 JP 2022023322
(71) Applicant: FUJIFILM Wako Pure Chemical Corporation, Osaka 540-8605 (JP)
(72) Inventor: MORITSUGU, Keiko, Tokyo 103-0023 (JP); WADA, Shogo, Osaka-shi, Osaka 540-8605 (JP); BABA, Sadaharu, Osaka-shi, Osaka 540-8605 (JP)
(74) Representative: Klunker IP Patentanwälte PartG mbB
(86) International application number: PCT/JP2023/005573
(87) International publication number: WO 2023/157937

(57) **Abstract**

A measuring device of the present disclosure includes a measurement part that measures a target substance contained in a sample solution, a tool placement area on which a tool used for the measurement is placed and that is disposed above the measurement part in a vertical direction, a dispensing part that dispenses a liquid and is movable above the tool placement area, and a housing that accommodates the measurement part, the tool placement area, and the dispensing part therein.

## Description

### BACKGROUND OF THE INVENTION

### 1. Field of the Invention

The technology of the present disclosure relates to a measuring device.

### 2. Description of the Related Art

A measuring device that measures a target substance contained in a liquid sample solution is known. As an example of the measuring device, there is a measuring device used for an endotoxin test to check whether or not a sample solution containing a reagent or the like contains endotoxin, which is a target substance. The endotoxin is a lipopolysaccharide present in a cell wall of a gram-negative bacterium, and is a pyrogen that induces a biological reaction such as fever in a case of entering a body. As the sample, there is a collected liquid obtained by collecting an attachment adhering to an outer surface of a medical instrument or the like. Here, the sample is a sample stock solution which is a measurement target. In addition, the sample solution includes not only a solution obtained by diluting the sample stock solution, but also a solution obtained by adding a reagent or the like to a solution obtained by diluting the sample stock solution, or a solution obtained by adding a reagent or the like to the sample stock solution. As the medical instrument or the like, there is a medical instrument to be inserted into the body, such as a catheter and an injection needle. The endotoxin test is a test in which a sample solution containing a sample is prepared and the amount of endotoxin contained in the prepared sample solution is measured, and is performed using a lysate reagent that produces agglutination and coagulation reactions with endotoxin. A lysate reagent prepared from a horseshoe crab hemocyte extract is commonly called LAL (short for *Limulus* Amebocyte Lysate). As a measuring device that measures the amount of endotoxin in the sample solution, for example, a device disclosed in JP2007-510911A is known.

JP2007-510911A discloses a device that performs an online test for the presence of endotoxin in a fluid sample. The device comprises a fluid extraction system that acquires a fluid sample from a fluid system line, an assembly including a fluid receiving member into which the fluid sample and a drug are introduced, and a detection system that determines the presence of endotoxin in the fluid receiving member.

### SUMMARY OF THE INVENTION

However, in the device disclosed in JP2007-510911A, a plurality of tip well housings having tips and a plurality of well plates as the fluid receiving member are disposed in a horizontal direction, which causes a problem in that the entire device is increased in size.

The present disclosure has been made in view of the circumstances, and an object of the present disclosure is to provide a measuring device that can be miniaturized as a whole.

A measuring device according to one aspect of the present disclosure comprises a measurement part that measures a target substance contained in a sample solution, a tool placement area on which a tool used for the measurement is placed and that is disposed above the measurement part in a vertical direction, a dispensing part that dispenses a liquid and is movable above the tool placement area, and a housing that accommodates the measurement part, the tool placement area, and the dispensing part therein.

In the measuring device according to the aspect, the tool may include a tip that is replaceably mounted on a distal end of the dispensing part, and the tool placement area may be a tip placement area on which the tip is placed.

In addition, in the measuring device according to the aspect, a tray that is movable in a horizontal direction between a storage position where the tray is stored inside the measurement part and a pull-out position where the tray is pulled out from the storage position to an outside, and on which a measurement plate accommodating the sample solution is replaceably disposed may be provided.

In addition, the measuring device according to the aspect may further comprise a dilution plate placement area on which a dilution plate used for diluting a liquid is placed, and the dilution plate placement area may be provided below the tip placement area in the vertical direction and above the tray in the vertical direction.

In addition, in the measuring device according to the aspect, the dilution plate placement area may be provided at a position that does not overlap the tray in the pull-out position in plan view.

In addition, the measuring device according to the aspect may further comprise an upper opening and a lower opening that are formed on one surface of the housing and are provided at different positions in the vertical direction, an upper cover for opening and closing the upper opening, and a lower cover for opening and closing the lower opening, and the upper cover may move between an open position for opening the upper opening and a closed position for closing the upper opening and covering the tip placement area, and the lower cover may move between an open position for opening the lower opening and a closed position for closing the lower opening.

In addition, the measuring device according to the aspect may further comprise a specimen rack that is disposed in the housing and stores a specimen container for accommodating a sample stock solution contained in the sample solution, and the upper cover may cover the specimen rack in the closed position.

In addition, in the measuring device according to the aspect, the specimen rack may be capable of being pulled out to an outside of the housing through the upper opening.

In addition, in the measuring device according to the aspect, the lower cover may be configured to cover the measurement part disposed below the tip placement area in the vertical direction and to be opened forward on an upper portion side by a hinge provided on a lower portion side with respect to the housing, and the upper cover may be configured to be opened by moving to an upper side in the vertical direction with respect to the housing.

In addition, in the measuring device according to the aspect, the target substance may be endotoxin.

According to the technology of the present disclosure, it is possible to provide a measuring device that can be miniaturized as a whole.

### BRIEF DESCRIPTION OF THE DRAWINGS

Fig. 1 is a perspective view showing an appearance of a measuring device according to a first embodiment.
Fig. 2 is a perspective view showing the appearance of the measuring device according to the first embodiment in a state in which an upper cover and a lower cover are opened.
Fig. 3 is a schematic perspective view showing a configuration inside a housing in the measuring device according to the first embodiment.
Fig. 4 is an enlarged perspective view showing a configuration of a lower part inside the housing in the measuring device according to the first embodiment.
Fig. 5 is a plan view showing the configuration of the lower part inside the housing in the measuring device according to the first embodiment.
Fig. 6 is a front view showing the configuration of the lower part inside the housing of the measuring device according to the first embodiment.
Fig. 7A is a front view showing a state in which a dispensing nozzle and a tip of the measuring device according to the first embodiment are separated from each other.
Fig. 7B is a front view showing a state in which the tip is mounted on the dispensing nozzle of the measuring device according to the first embodiment.
Fig. 8 is a flowchart showing each step of the measuring device according to the first embodiment.
Fig. 9 is a plan view showing a positional relationship between a tray on which a measurement plate is placed, a dilution plate placement area on which a dilution plate is placed, a tip placement area on which a tip is placed, and a specimen rack on which a specimen container is placed in the measuring device according to the first embodiment.
Fig. 10 is a perspective view of a part of the measuring device according to the first embodiment, which shows a state in which the measurement plate is placed on the tray pulled out to a pull-out position.
Fig. 11 is a perspective view of a part of the measuring device according to the first embodiment, which shows a state in which the tray on which the measurement plate is placed is moved to a storage position inside a measurement part.
Fig. 12 is a plan view showing a state in which the specimen rack is pulled out to an outside of the housing in the measuring device according to the first embodiment.

### DESCRIPTION OF THE PREFERRED EMBODIMENTS

Hereinafter, embodiments for implementing the technology of the present disclosure will be described. In the following description, a direction indicated by an arrow UP, which is appropriately shown in the drawing, is defined as an upper side in an up-down direction of a device. The up-down direction of the device coincides with a vertical direction in an example of the present disclosure. It should be noted that, in each drawing, components that are less relevant to the technology of the present disclosure are not shown.

A measuring device 10, is, for example, a device used for an endotoxin test to check whether or not a sample solution containing a reagent or the like contains endotoxin, which is a target substance.

As described above, the endotoxin is a lipopolysaccharide present in a cell wall of a gram-negative bacterium, and is a pyrogen that induces a biological reaction such as fever in a case of entering a body. As the sample, there is a collected liquid obtained by collecting an attachment adhering to an outer surface of a medical instrument or the like. As described above, the sample is a sample stock solution which is a measurement target. In addition, the sample solution includes not only a solution obtained by diluting the sample stock solution, but also a solution obtained by adding a reagent or the like to a solution obtained by diluting the sample stock solution, or a solution obtained by adding a reagent or the like to the sample stock solution. As the medical instrument or the like, there is a medical instrument to be inserted into the body, such as a catheter and an injection needle. The endotoxin test is a test in which a sample solution containing a sample is prepared and the amount of endotoxin contained in the prepared sample solution is measured, and is performed using a lysate reagent that produces agglutination and coagulation reactions with endotoxin. A lysate reagent prepared from a horseshoe crab hemocyte extract is commonly called LAL (short for *Limulus* Amebocyte Lysate).

In addition, as an example, the measuring device 10 uses an LAL reagent as a lysate reagent to perform endotoxin measurement by a turbidimetric method using, as an indicator, a change in turbidity of the sample solution in a process of gelation of the lysate reagent by a reaction with endotoxin.

### [External Configuration of Measuring Device]

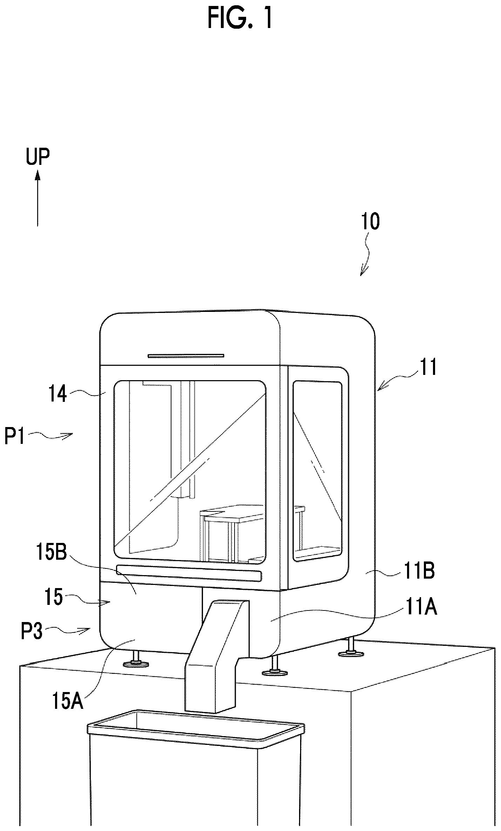

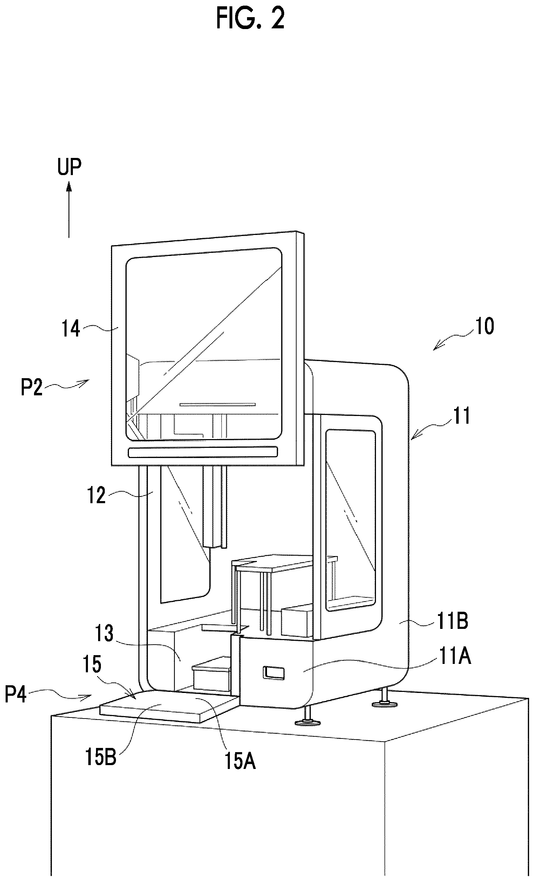

Fig. 1 is a perspective view showing an appearance (that is, an external configuration) of the measuring device 10 according to a first embodiment of the present disclosure. In addition, Fig. 2 is a perspective view showing a state in which an upper cover and a lower cover are moved to an open position in the measuring device 10.

As shown in Figs. 1 and 2, the measuring device 10 comprises a housing 11, an upper opening 12 and a lower opening 13 formed on a front surface 11A as one surface of the housing 11, an upper cover 14 capable of opening and closing the upper opening 12, and a lower cover 15 capable of opening and closing the lower opening 13.

The upper opening 12 and the lower opening 13 are provided at different positions in a vertical direction of the housing 11. For example, the upper opening 12 is provided on an upper portion side in the vertical direction of the front surface 11A of the housing 11 and extends over almost the entire region in a width direction of the front surface 11A of the housing 11. In addition, for example, the lower opening 13 is provided on a lower side in the vertical direction of the front surface 11A of the housing 11 and on one side in the width direction of the front surface 11A of the housing 11. As an example, the upper opening 12 and the lower opening 13 are connected in the vertical direction of the housing 11, but the upper opening 12 and the lower opening 13 may be separately formed.

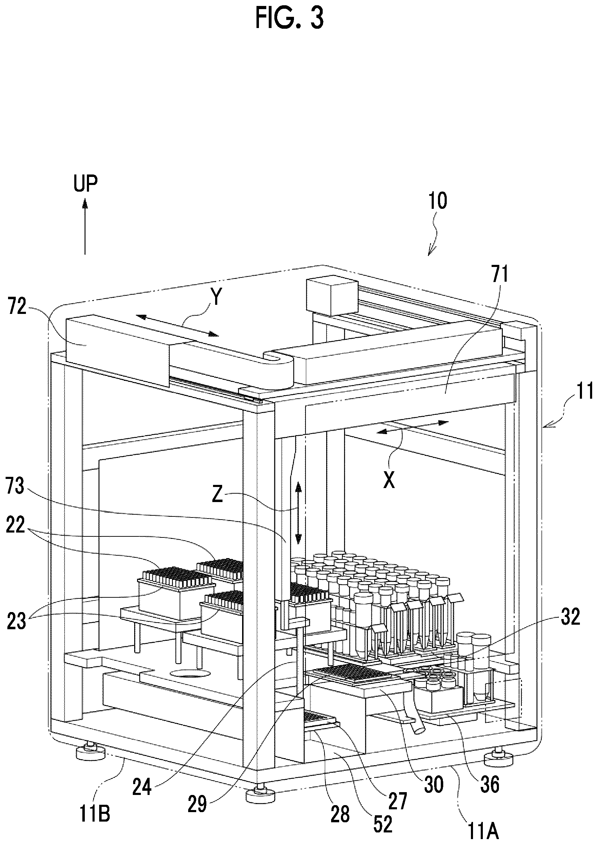

The upper cover 14 moves between an open position P2 for opening the upper opening 12 and a closed position P1 for closing the upper opening 12. In the present example, the upper cover 14 is moved to the open position P2 on the upper side in the vertical direction with respect to the housing 11 to open the upper opening 12 of the housing 11 (see Fig. 2). The upper cover 14 covers, for example, a tip placement area 23 and a specimen rack 32 (see Fig. 3 and the like), which will be described below, in the closed position P1. Here, the fact that the upper cover 14 covers the tip placement area 23 and the specimen rack 32 means that in a case where the upper cover 14 is projected horizontally onto a vertical plane, projection of the upper cover 14 includes projection of the tip placement area 23 and the specimen rack 32.

The upper cover 14 may be formed of a transparent material. In addition, a part of the housing 11 may be formed of a transparent material.

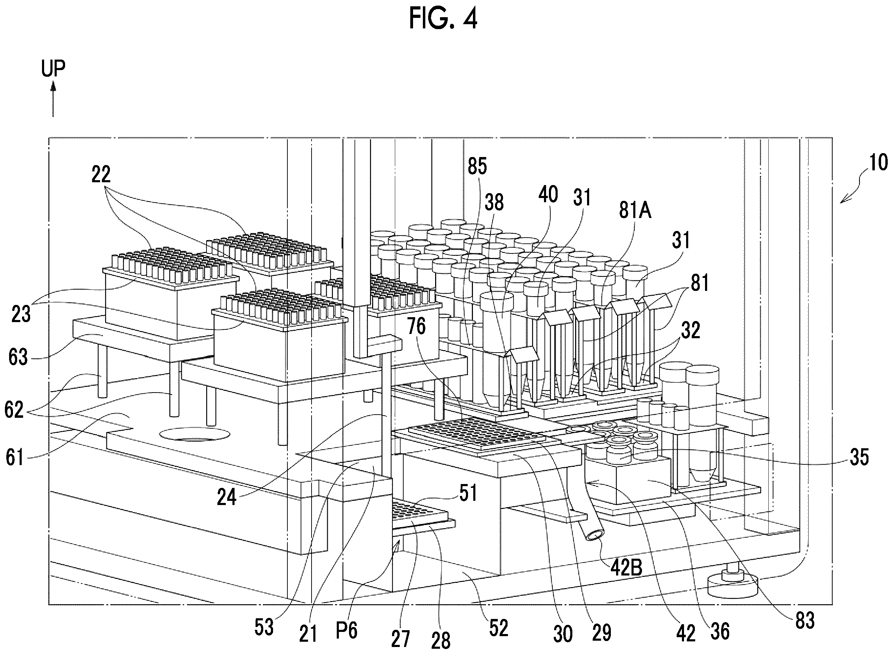

The lower cover 15 moves between an open position P4 for opening the lower opening 13 and a closed position P3 for closing the lower opening 13. In the example of the present disclosure, the lower cover 15 covers, in the closed position P3, a measurement part 21 (see Fig. 4 and the like) disposed below the tip placement area 23 in the vertical direction. Here, the fact that the lower cover 15 covers the measurement part 21 means that in a case where the lower cover 15 is projected horizontally onto a vertical plane, projection of the lower cover 15 includes projection of the measurement part 21. The lower cover 15 is opened forward on an upper portion 15B side by a hinge (not shown) provided on a lower portion 15A side with respect to the housing 11.

### [Overall Configuration of Measuring Device]

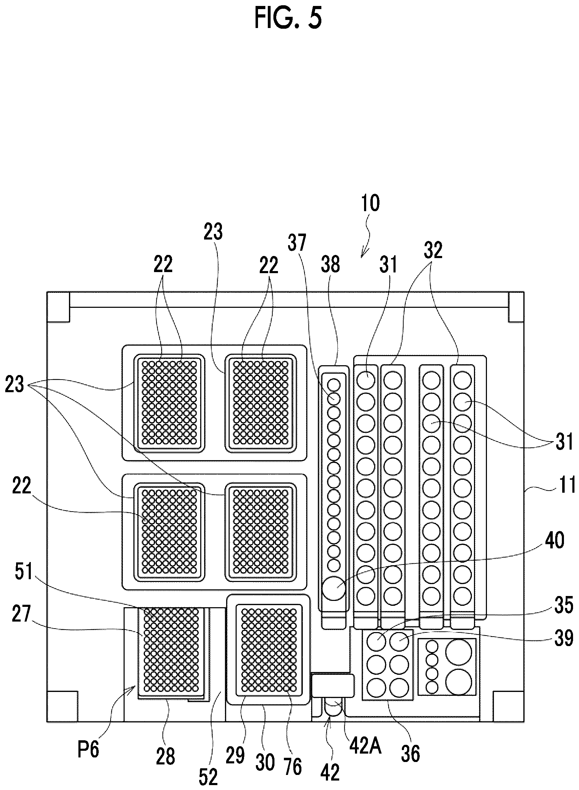

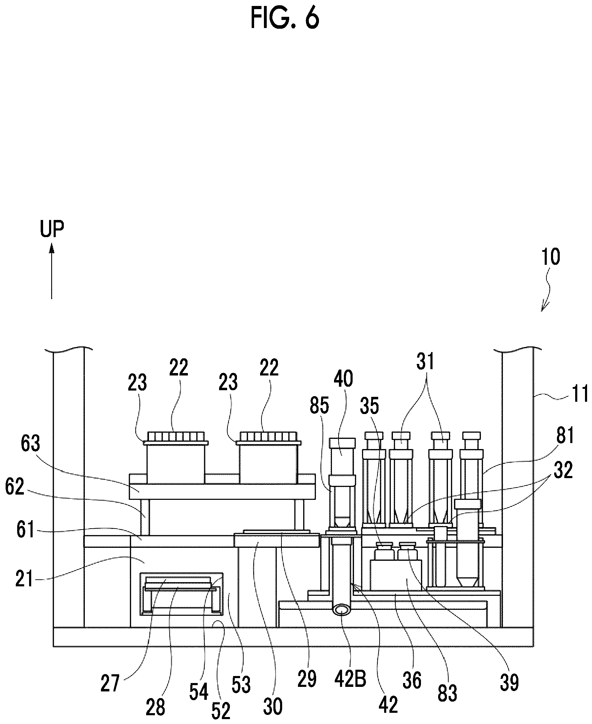

Fig. 3 is a perspective view showing an internal configuration of the measuring device 10, and Fig. 4 is an enlarged perspective view showing an internal configuration of a lower part of the measuring device 10. In addition, Fig. 5 is a plan view showing a lower part of the housing 11 of the measuring device 10, and Fig. 6 is a front view showing the lower part of the measuring device 10. In Fig. 3 and the like, a direction in which a dispensing nozzle 24 moves in the width direction of the housing 11 (for example, a direction along the front surface 11A) is defined as an X direction, and a direction in which the dispensing nozzle 24 moves in a depth direction of the housing 11 that intersects the X direction (for example, a direction along the side surface 11B) is defined as a Y direction. In addition, a direction in which the dispensing nozzle 24 moves in a vertical direction orthogonal to the X direction and the Y direction is defined as a Z direction.

As shown in Figs. 3 to 6, the measuring device 10 comprises a measurement part 21 that measures a target substance contained in a sample solution, a tip placement area 23 on which a tip 22 as an example of a tool used for the measurement is placed, and a dispensing nozzle 24 that dispenses a liquid. The tip placement area 23 is an example of a tool placement area. The dispensing nozzle 24 is an example of a dispensing part. The measurement part 21, the tip placement area 23, and the dispensing nozzle 24 are accommodated inside the housing 11.

The measuring device 10 comprises a tray 28 on which a measurement plate 27 accommodating the sample solution is replaceably disposed, and a dilution plate placement area 30 on which a dilution plate 29 used for diluting a liquid is placed. The dilution plate 29 is a well plate in which a plurality of small wells 76 are provided in a matrix. The dilution plate 29 is used for preparation of various liquids, such as dilution of a liquid. In addition, the measuring device 10 comprises a specimen rack 32 in which a specimen container 31 for accommodating a sample stock solution contained in the sample solution is disposed. The tray 28, the dilution plate placement area 30, and the specimen rack 32 are accommodated inside the housing 11.

In addition, the measuring device 10 comprises a first container placement area 36 in which a standard solution container 35 accommodating a standard stock solution is disposed, and a second container placement area 38 in which a dilution container 37 for diluting a standard solution is disposed. The standard stock solution is a liquid used as a reference in a case of measuring a concentration of endotoxin in the sample solution, and is a liquid containing standard endotoxin with a known concentration. The standard solution is a liquid obtained by diluting the standard stock solution with water for test. In a case of measuring the sample solution, a plurality of types of standard solutions with different concentrations are prepared in order to acquire a calibration curve during the measurement.

In addition, as an example, a reagent container 39 for accommodating a lysate reagent is disposed in the first container placement area 36. In addition, as an example, a water container 40 accommodating water for test is disposed in the second container placement area 38. The first container placement area 36 and the second container placement area 38 are accommodated inside the housing 11.

Further, the measuring device 10 comprises a disposal part 42 where the used tip 22 is disposed of. An insertion port 42A of the disposal part 42 is disposed inside the housing 11, and a discharge port 42B side of the disposal part 42 is pulled out to an outside of the housing 11.

### (Measurement Part and Tray)

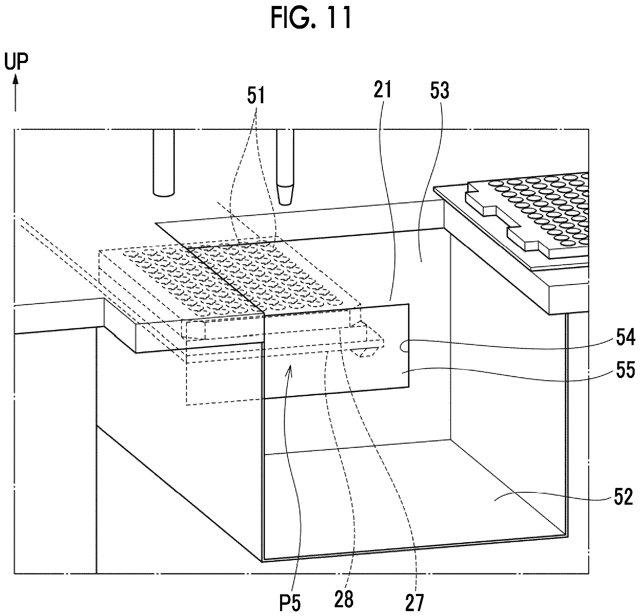

As shown in Figs. 3 to 6, the measurement part 21 is provided with the tray 28 on which the measurement plate 27 is replaceably disposed as described above. The measurement part 21 measures the target substance contained in the sample solution accommodated in the measurement plate 27 in a state in which the measurement plate 27 disposed on the tray 28 is disposed at a storage position P5 (see Fig. 11) inside the measurement part 21.

The measurement part 21 optically measures the target substance in the sample solution accommodated in the measurement plate 27. The measurement part 21 is provided with a light source (not shown) used for the measurement, a light intensity sensor (not shown) such as a photodiode, and the like. In the present example, the endotoxin concentration is measured using, for example, a method called a turbidimetric method. The turbidimetric method is a method using, as an indicator, a change in turbidity in a process of gelation of the lysate reagent due to the action of endotoxin. The turbidity of the sample solution changes depending on the amount of endotoxin contained in the sample solution and the elapsed time after the lysate reagent is added. In a case where the turbidity of the sample solution changes, the light intensity of measurement light transmitted through the sample solution changes. Therefore, the state and the transition of the turbidity of the sample solution can be measured by measuring the change over time in the amount of transmitted light by the light intensity sensor provided in the measurement part 21. The measuring device 10 is provided with a calculation unit (not shown). The calculation unit calculates the amount of endotoxin in the sample solution based on the state and the transition of the turbidity of the sample solution measured by the measurement part 21.

The measurement plate 27 is a well plate in which a plurality of small wells 51 are provided in a matrix, and a sample solution, which is a measurement target, is accommodated in an inside of the well 51.

As described above, the measurement part 21 is disposed below the tip placement area 23 in the vertical direction. As an example, the measurement part 21 and the tip placement area 23 are disposed along one side surface 11B (see Fig. 3) of the side surfaces 11B (see Fig. 1) on both sides of the housing 11 in the width direction. The measurement part 21 and the tip placement area 23 are disposed on a back side in the depth direction (that is, a back side in the Y direction) of the housing 11.

A recess-shaped portion 52 that is recessed to a lower side in the vertical direction is provided on a front surface 11A side of the housing 11 in the measurement part 21. A wall portion 53 on the front surface 11A side in the measurement part 21 is disposed at a position facing the recess-shaped portion 52. An opening 54 into which the tray 28 is inserted is provided in the wall portion 53 of the measurement part 21 (see Fig. 6).

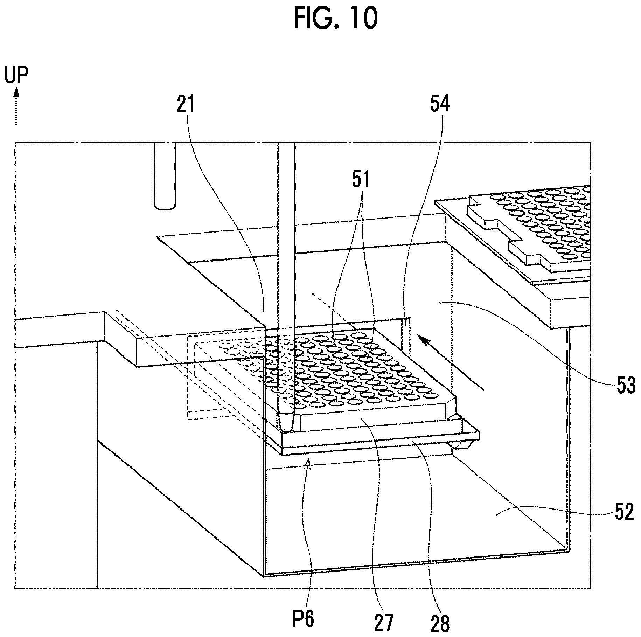

The tray 28 is movable between a storage position P5 (see Fig. 11) where the tray 28 is stored inside the measurement part 21 and a pull-out position P6 (see Fig. 4 and Fig. 10) where the tray 28 is pulled out from the storage position P5 to an outside of the measurement part 21. In side view (that is, in a state in which the tray 28 is viewed from the front surface 11A of the housing 11 shown in Fig. 6), the tray 28 is provided at a position overlapping the measurement part 21. The tray 28 moves in a horizontal direction between the pull-out position P6 and the storage position P5. Although not shown, a support portion that supports the tray 28 to be slidable in the horizontal direction and an actuator that moves the tray 28 are provided inside the measurement part 21. As a result, the tray 28 moves in the horizontal direction between the pull-out position P6 and the storage position P5.

### (Tip Placement Area)

As shown in Figs. 3 to 6, the tool used for the measurement includes a tip 22 that is replaceably mounted on a distal end of the dispensing nozzle 24. A plurality of the tips 22 are placed on the tip placement area 23. The number of the tips 22 is large because the tips 22 are single-use for the purpose of preventing contamination. Therefore, the number and variety of the tools also tend to increase, and there are many merits to reducing a horizontal occupied area of the tip placement area 23.

In the example of the present disclosure, four tip placement areas 23 are disposed, and a plurality of tips 22 are placed in each of the tip placement areas 23. The tip placement area 23 is disposed in two rows in a lateral direction shown in Fig. 5 and in two rows in a longitudinal direction shown in Fig. 5 in plan view (see Fig. 5). The plurality of tips 22 are arranged in the tip placement area 23 in the lateral direction and the longitudinal direction shown in Fig. 5. The plurality of tips 22 are inserted into recesses of the tip placement area 23 from above.

The tip placement area 23 is disposed above the measurement part 21 in the vertical direction (see Figs. 3, 4, and 6). That is, the tip placement area 23 is disposed on the back side in the depth direction (that is, the Y direction) along the side surface 11B of the housing 11. As an example, the tip placement area 23 is provided on an upper part of a support plate 63 supported on an upper part of a plurality of rods 62 bonded to a plate material 61 disposed on an upper surface of the measurement part 21.

### (Dispensing Nozzle)

The dispensing nozzle 24 is a cylindrical member disposed in the vertical direction. The dispensing nozzle 24 is movable above the tip placement area 23. In the example of the present disclosure, as described above, the dispensing nozzle 24 is movable in the X direction along the front surface 11A of the housing 11, in the Y direction along the side surface 11B of the housing 11, which is orthogonal to the X direction, and in the Z direction orthogonal to the X direction and the Y direction (see Fig. 3). In the examples of the present disclosure, the Z direction coincides with the vertical direction.

As shown in Fig. 3, the measuring device 10 comprises an X-direction drive unit 71 that moves the dispensing nozzle 24 in the X direction along the front surface 11A of the housing 11, and a Y-direction drive unit 72 that moves the dispensing nozzle 24 in the Y direction along the side surface 11B of the housing 11. Further, the measuring device 10 comprises a Z-direction drive unit 73 that moves the dispensing nozzle 24 in the Z direction along the vertical direction of the housing 11. The dispensing nozzle 24 is movable to any position above the tip placement area 23, the tray 28, the dilution plate placement area 30, the first container placement area 36, and the second container placement area 38 by the X-direction drive unit 71, the Y-direction drive unit 72, and the Z-direction drive unit 73.

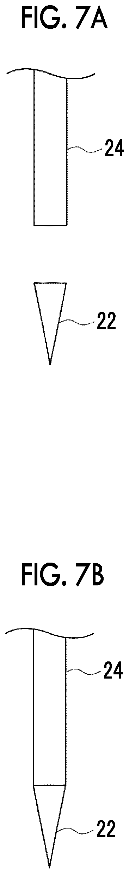

The tip 22 is replaceably mounted on the distal end of the dispensing nozzle 24. As shown in Fig. 7A, the dispensing nozzle 24 is moved above any one tip 22 of the plurality of tips 22 placed on the tip placement area 23. Then, the dispensing nozzle 24 is lowered to mount one tip 22 on the distal end of the dispensing nozzle 24 as shown in Fig. 7B. For example, the tip 22 may be mounted on the distal end of the dispensing nozzle 24 by engaging an engaged portion of the dispensing nozzle 24 with an engaging portion provided on the tip 22. In a state in which the tip 22 is mounted on the dispensing nozzle 24, the dispensing nozzle 24 can use the tip 22 to suck a liquid from one container or can discharge a liquid from the tip 22 to another container. As a result, the dispensing nozzle 24 can dispense the liquid by using the tip 22.

As shown in Fig. 4, the dispensing nozzle 24 is movable above the measurement plate 27 disposed on the tray 28 in a state in which the tray 28 is pulled out to the pull-out position P6. In a state in which the tip 22 is mounted on the dispensing nozzle 24, the dispensing nozzle 24 can dispense the sample solution into the wells 51 of the measurement plate 27.

### (Dilution Plate Placement Area)

As shown in Figs. 3 to 6, the dilution plate placement area 30 is provided below the tip placement area 23 in the vertical direction and above the tray 28 in the vertical direction. The dilution plate placement area 30 is provided at a position that does not overlap the tray 28 in the pull-out position P6 in plan view. In the example of the present disclosure, the dilution plate placement area 30 is provided at a position adjacent to the recess-shaped portion 52 on the front side of the measurement part 21 in the device depth direction (Y direction) in plan view.

For example, the dispensing nozzle 24 moves above the dilution plate placement area 30. Then, a sample solution can be prepared by injecting the sample stock solution and the diluent into the well 76 of the dilution plate 29 on the dilution plate placement area 30 using the dispensing nozzle 24. Further, the dispensing nozzle 24 moves to the tray 28 in the pull-out position P6. The prepared sample solution can be injected into the measurement plate 27 on the tray 28.

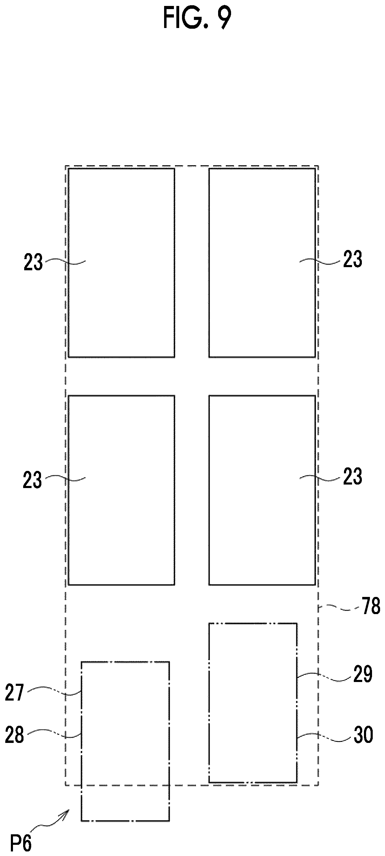

As shown in Fig. 9, the tip placement area 23, the dilution plate placement area 30, and the tray 28 are each formed of a rectangular member in plan view. In the example of the present disclosure, at least a part of the tray 28 in the pull-out position P6 is included in an imaginary rectangular shape 78 that circumscribes the tip placement area 23 and the dilution plate placement area 30 in plan view.

### (Specimen Rack)

As shown in Figs. 3 to 6, a plurality of specimen containers 31 for accommodating a sample stock solution are stored in the specimen rack 32. The specimen container 31 is, for example, a test tube. As an example, four specimen racks 32 are provided in the width direction (that is, the X direction) of the housing 11. In plan view, the specimen rack 32 has an elongated shape in the depth direction (that is, the Y direction) of the housing 11, and four specimen racks 32 are disposed side by side in the width direction (that is, the X direction) of the housing 11. The specimen rack 32 stores a plurality of specimen containers 31 arranged in a row. The plurality of specimen containers 31 are supported in a state of being inserted into a plurality of openings 81A of a support frame 81 provided on an upper portion of the specimen rack 32, respectively (see Fig. 4).

The four specimen racks 32 are disposed on the back side in the depth direction (that is, the Y direction) of the housing 11. In addition, the four specimen racks 32 are disposed on a side opposite to the tip placement area 23 in the width direction (that is, the X direction) of the housing 11.

A lower end part of the specimen rack 32 is disposed to be aligned with the dilution plate placement area 30 in the horizontal direction. An upper end part of the specimen rack 32 is disposed on a side of the tip placement area 23. In a state in which the specimen containers 31 are stored in the specimen rack 32, upper end parts of the specimen containers 31 is disposed to be aligned with an upper end part of the tip placement area 23 in the horizontal direction.

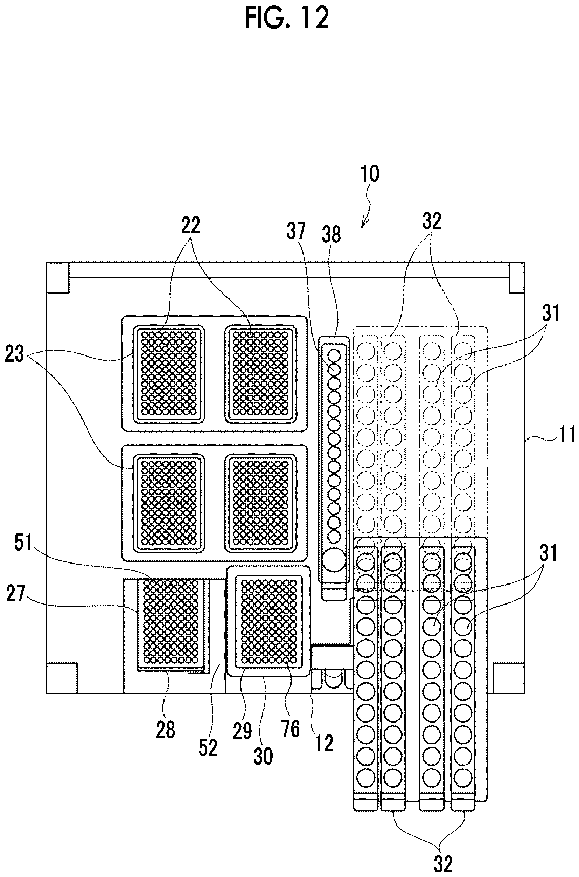

As shown in Fig. 12, the specimen rack 32 can be pulled out to the outside of the housing 11 through the upper opening 12 (see Fig. 2) of the housing 11. Although not shown, as an example, a rail that supports the specimen rack 32 to be slidable is provided on a lower side of the specimen rack 32 inside the housing 11. As a result, the specimen rack 32 is pulled out to the outside along the rail through the upper opening 12 (see Fig. 2) of the housing 11.

### (First Container Placement Area)

As shown in Figs. 3 to 6, the first container placement area 36 is located on a side opposite to the recess-shaped portion 52 in the width direction (that is, the X direction) of the housing 11 on the front surface 11A side of the housing 11. In plan view, the first container placement area 36 is provided at a position that does not overlap the dilution plate placement area 30. A lower end part of the first container placement area 36 is disposed to be aligned with the tray 28 in the horizontal direction. The first container placement area 36 is provided with a support portion 83 comprising an opening for supporting the standard solution container 35 or the reagent container 39.

### (Second Container Placement Area)

As shown in Figs. 3 to 6, the second container placement area 38 is disposed between the tip placement area 23 and the specimen rack 32 on the back side in the depth direction (that is, the Y direction) of the housing 11. A lower end part of the second container placement area 38 is disposed to be aligned with the lower end part of the specimen rack 32 in the horizontal direction. The second container placement area 38 is provided with a support portion 85 comprising an opening for supporting the dilution container 37 or the water container 40. Although not shown, the second container placement area 38 has a rack shape and can be pulled out to the outside of the housing 11 in the same manner as the specimen rack 32. As an example, a rail (not shown) that supports the second container placement area 38 to be slidable is provided on a lower side of the second container placement area 38 inside the housing 11.

### (Disposal Part)

The disposal part 42 is provided in a central portion in the width direction (that is, the X direction) of the housing 11 on the front surface 11A side of the housing 11. The disposal part 42 is disposed between the dilution plate placement area 30 and the first container placement area 36. The insertion port 42A of the disposal part 42 is disposed on a side of the dilution plate placement area 30 so as to be aligned with the dilution plate placement area 30 in the horizontal direction. The used tip 22 is inserted into the disposal part 42 from the insertion port 42A and is disposed of from the discharge port 42B to the outside of the housing 11.

### (Measurement Process)

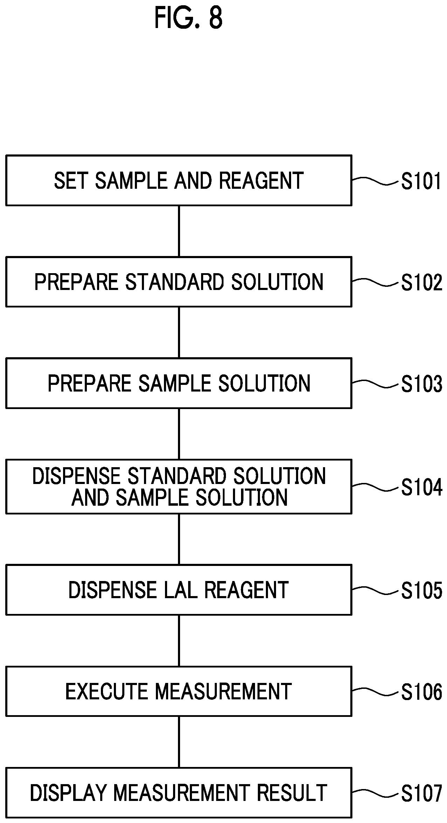

Next, a measurement process executed by the measuring device 10 will be described. Fig. 8 is a flowchart showing an example of each step of the measurement process.

In Fig. 8, measurement of step S106 is a step of measuring the sample solution accommodated in the measurement plate 27 by the measurement part 21, and step S101 to step S105 are steps of preparing a liquid such as the sample solution accommodated in the measurement plate 27.

As shown in Fig. 8, in step S101, a sample and a reagent are set in the measuring device 10. The reagent is a lysate reagent. The setting of the sample and the reagent is executed in a state in which the upper cover 14 is moved to the open position P2 for opening the upper opening 12 of the housing 11 (see Fig. 2).

First, the plurality of specimen containers 31 for accommodating a sample stock solution are stored in the specimen rack 32 (see Fig. 5). As a result, the sample is set in the measuring device 10. For example, as shown in Fig. 12, the specimen containers 31 are stored in the specimen rack 32 in a state in which the specimen rack 32 is pulled out to the outside of the housing 11 through the upper opening 12 (see Fig. 2) of the housing 11. Thereafter, the specimen rack 32 is moved to a preset position in the housing 11.

In addition, the reagent container 39 for accommodating a lysate reagent is disposed in the first container placement area 36. As a result, the reagent is set in the measuring device 10. In addition, although not shown in Fig. 8, for example, the standard solution container 35 accommodating a standard stock solution is disposed in the first container placement area 36. In addition, the water container 40 accommodating water for test is disposed in the second container placement area 38.

After the setting of the sample and the reagent is completed, the upper cover 14 is moved to the closed position P1 for closing the upper opening 12 (see Fig. 1).

Each of steps S102 to S105 is a step of preparing various liquids including the sample solution used for the measurement. Each of these steps is performed by the dispensing nozzle 24 operating in a preset procedure in the tip placement area 23, the first container placement area 36, or the second container placement area 38.

Step S102 is a step of preparing a standard solution. The standard solution is prepared in the dilution container 37 in the second container placement area 38. The dispensing nozzle 24 prepares standard solutions with a plurality of concentrations by diluting the standard stock solution with water for test in the dilution container 37 while moving between the standard solution container 35 and the water container 40. The reason for preparing the standard solutions with different concentrations is to acquire a calibration curve that serves as a reference in a case of measuring the concentration of the sample solution. The dispensing nozzle 24 is moved to the tip placement area 23 and replaces the tip 22 each time a different liquid is sucked and discharged. The used tip 22 is disposed of in the disposal part 42.

Step S103 is a step of preparing a sample solution. The sample solution is prepared in the dilution plate 29 disposed in the dilution plate placement area 30. The dispensing nozzle 24 prepares a sample solution by diluting the sample stock solution with water for test in the dilution plate 29 while moving between the specimen container 31 and the water container 40. In the step of preparing the sample solution as well, the replacement and disposal of the tip 22 are repeatedly performed.

Next, in step S104, the prepared standard solution and sample solution are dispensed onto the measurement plate 27. The measurement plate 27 is disposed on the tray 28 in the pull-out position P6 (see Fig. 10). The dispensing nozzle 24 dispenses the sample solution prepared in the dilution plate 29 into the wells 51 of the measurement plate 27. Further, the dispensing nozzle 24 dispenses the standard solutions with a plurality of concentrations prepared in the dilution container 37 into the wells 51 of the measurement plate 27. In addition, in addition to a mixed solution obtained by mixing the standard solution and the sample solution, water for test is also dispensed into the wells 51 of the measurement plate 27. As a result, the standard solution, the sample solution, the mixed solution obtained by mixing the sample solution and the standard solution, and the water for test are dispensed into the different wells 51 of the measurement plate 27.

In step S105, an LAL reagent as the lysate reagent is dispensed into each of the wells into which the standard solution, the sample solution, and the mixed solution have been dispensed. The LAL reagent is dispensed by the dispensing nozzle 24 sucking the LAL reagent from the reagent container 39 and discharging the LAL reagent into each well 51 of the measurement plate 27.

As described above, in step S 102 to step S105, the dispensing nozzle 24 prepares various liquids used for the measurement while moving to each part in the measuring device 10, and dispenses the prepared liquids onto the measurement plate 27.

After that, in step S106, the measurement is executed. Since the measurement plate 27 is disposed on the tray 28, as shown in Fig. 11, the measurement plate 27 is stored in the measurement part 21 by moving the tray 28 to the storage position P5 inside the measurement part 21 through the opening 54 of the measurement part 21. In this state, a lid 55 of the opening 54 of the measurement part 21 is closed, and the measurement is performed. For example, in the measurement part 21, the turbidity of a reaction solution obtained by adding the LAL reagent to the sample solution, the standard solution, or the like in each well 51 in the measurement plate 27 is optically measured according to the procedure of the turbidimetric method, and the endotoxin concentration is measured by performing calculation based on a measurement result.

Next, in step S107, the measurement result is displayed. Although not shown, for example, a personal computer is electrically connected to the measuring device 10, and the measurement result is displayed on a display unit of the personal computer. As a result, each step of the measuring device 10 is completed.

### [Action and Effect]

Next, the action and the effect of the first embodiment will be described.

The measuring device 10 comprises the measurement part 21 that measures a target substance contained in a sample solution, the tip placement area 23 on which the tip 22 used for the measurement is placed, the dispensing nozzle 24 that dispenses a liquid, and the housing 11 that accommodates the measurement part 21, the tip placement area 23, and the dispensing nozzle 24 therein. The tip placement area 23 is disposed above the measurement part 21 in the vertical direction. In addition, the dispensing nozzle 24 is movable above the tip placement area 23.

In the measuring device 10, the tip placement area 23 is disposed above the measurement part 21 in the vertical direction as described above. As a result, the horizontal occupied area can be reduced as compared with a case where the tip placement area 23 and the measurement part 21 are disposed at different positions in the horizontal direction, and the device can be miniaturized as a whole. In addition, since the dispensing nozzle 24 that dispenses the sample solution moves above the tip placement area 23, the tip placement area 23 needs to be disposed above the measurement part 21 in order to ensure the operation of the dispensing nozzle 24.

In addition, as tools, the tip 22 that is replaceably mounted on the distal end of the dispensing nozzle 24, the measurement plate 27 for preparing the sample solution, and the like are required. The number of the tips 22 is large because the tips 22 are single-use for the purpose of preventing contamination. Therefore, the number and variety of the tools also tend to increase, and there are many merits to reducing the horizontal occupied area of the tip placement area 23.

In addition, in the measuring device 10, the tool includes the tip 22 that is replaceably mounted on the distal end of the dispensing nozzle 24, and the tip placement area 23 on which the tip 22 is placed is provided.

For example, the tip placement area 23 on which the tip 22 is placed has a shorter length in the vertical direction than that of a test tube or the like in many cases. Therefore, the tip placement area 23 is disposed above the measurement part 21 in the vertical direction, so that a space can be saved. In addition, a height of the measuring device 10 can be reduced as compared with a case where another tool placement area such as the test tube is disposed above the measurement part.

In addition, the measuring device 10 is provided with the tray 28, and the tray 28 is movable in the horizontal direction between the storage position P5 where the tray 28 is stored inside the measurement part 21 and the pull-out position P6 where the tray 28 is pulled out from the storage position P5 to the outside. The measurement plate 27 is replaceably disposed on the tray 28, and the measurement plate 27 is provided with the wells 51 accommodating the sample solution.

As a result, the tray 28 is moved from the pull-out position P6 to the storage position P5 inside the measurement part 21, whereby the measurement plate 27 disposed on the tray 28 is stored in the storage position P5 inside the measurement part 21. Therefore, in the measuring device 10, a space required for installing an arm for moving the plate can be saved, and the device can be miniaturized as a whole.

In addition, in the measuring device 10, the dilution plate placement area 30 on which the dilution plate 29 used for diluting a liquid is placed is provided. The dilution plate placement area 30 is provided below the tip placement area 23 in the vertical direction and above the tray 28 in the vertical direction.

As a result, in a case of diluting the sample stock solution or the like by mounting the tip 22 placed on the tip placement area 23 on the dispensing nozzle 24 and moving the dispensing nozzle 24 and the tip 22 to the dilution plate 29 placed on the dilution plate placement area 30, a moving distance of the dispensing nozzle 24 and the tip 22 is shortened. Therefore, a total measurement time is shortened by shortening a movement time of the dispensing nozzle 24 and the tip 22.

In addition, in the measuring device 10, the dilution plate placement area 30 is provided at a position that does not overlap the tray 28 in the pull-out position P6 in plan view.

Therefore, in the measuring device 10, in a case where a liquid is diluted in the dilution plate 29 placed on the dilution plate placement area 30, there is no interference with the tray 28 in the pull-out position P6, and operability is improved.

In addition, the measuring device 10 is provided with the upper opening 12 and the lower opening 13 formed on the front surface 11A of the housing 11, the upper cover 14 capable of opening and closing the upper opening 12, and the lower cover 15 capable of opening and closing the lower opening 13. The upper opening 12 and the lower opening 13 are provided at different positions in the vertical direction. The upper cover 14 moves between the open position P2 for opening the upper opening 12 and the closed position P1 for closing the upper opening 12. The upper cover 14 covers the tip placement area 23 in a case where the upper cover 14 is moved to the closed position P1. In addition, the lower cover 15 moves between the open position P4 for opening the lower opening 13 and the closed position P3 for closing the lower opening 13.

Since the housing 11 is provided with the upper cover 14 that covers the tip placement area 23 at the closed position P1, the tip 22 can be disposed on the tip placement area 23 or the tip 22 in the tip placement area 23 can be taken out by moving the upper cover 14 to the open position P2. In addition, since the upper cover 14 covers the tip placement area 23 at the closed position P 1, contamination of the tip 22 placed on the tip placement area 23 can be suppressed. Further, since the housing 11 is provided with the lower cover 15, the lower cover 15 can cover the tray 28 or the measurement part 21 below the tip placement area 23 in the vertical direction.

In addition, the measuring device 10 is provided with the specimen rack 32 that is disposed in the housing 11 and that stores the specimen container 31 for accommodating a sample stock solution contained in the sample solution. The upper cover 14 covers the specimen rack 32 in the closed position P1.

Therefore, contamination of the sample stock solution of the specimen container 31 stored in the specimen rack 32 can be suppressed by the upper cover 14 moved to the closed position P1. In addition, the specimen container 31 can be disposed on the specimen rack 32 or the specimen container 31 can be taken out from the specimen rack 32 by moving the upper cover 14 to the open position P2.

In addition, in the measuring device 10, the specimen rack 32 can be pulled out to the outside of the housing 11 through the upper opening 12.

Therefore, in the measuring device 10, the specimen rack 32 is pulled out to the outside of the housing 11, so that it is easy to dispose the specimen container 31 on the specimen rack 32 or to take out the specimen container 31 from the specimen rack 32.

In addition, in the measuring device 10, the second container placement area 38 can be pulled out to the outside of the housing 11, so that it is easy to dispose the dilution container 37 or the like on the second container placement area 38 or to take out the dilution container 37 or the like from the second container placement area 38.

In addition, in the measuring device 10, the lower cover 15 covers the measurement part 21 disposed below the tip placement area 23 in the vertical direction. The lower cover 15 is opened forward on the upper portion 15B side by the hinge (not shown) provided on the lower portion 15A side with respect to the housing 11. The upper cover 14 is opened by moving to the upper side in the vertical direction with respect to the housing 11.

In the measuring device 10, the lower cover 15 is opened forward on the upper portion 15B side by the hinge provided on the lower part side of the housing 11, so that the lower cover 15 is less likely to interfere with the operation of the measurement part 21 in a case where the lower cover 15 is opened. In addition, the upper cover 14 is opened by moving to the upper side in the vertical direction with respect to the housing 11, so that it is not necessary to ensure a space for opening the upper cover 14 in the width direction of the measuring device 10.

In addition, in the measuring device 10, the target substance for the measurement is endotoxin.

In a case of the measuring device 10 used for the endotoxin test, in addition to preparation of a plurality of sample solutions with different concentrations, it is also necessary to prepare a plurality of standard solutions with different concentrations in order to acquire a calibration curve. Therefore, the number of the tips 22 and the number of the tip placement areas 23 tend to be larger than those of a measuring device for applications other than the endotoxin test. Therefore, there is a strong need to reduce the size of the housing 11 in the horizontal direction.

### [Modification Example]

The embodiment is an example, and various modifications can be made as shown below. In the embodiment, the sample stock solution is diluted and measured by the measurement part 21, but the technology of the present disclosure is not limited to this configuration. For example, a sample solution to which a reagent or the like is added without diluting the sample stock solution may be measured by the measurement part 21.

In addition, the lysate reagent used for the endotoxin measurement is not limited to the LAL reagent, and a TAL (short for *Tachypleus* Amebocyte Lysate) reagent prepared from a hemocyte extract of horseshoe crab *(Tachypleus tridentatus)* different from American horseshoe crab may be used.

In addition, the method of the endotoxin test is not limited to the turbidimetric method described in the embodiment, and a colorimetric method using color development by hydrolysis of a synthetic substrate as an indicator may be used.

In addition, the measurement using the horseshoe crab hemocyte extract is not limited to the endotoxin measurement, and may also be a β-glucan measurement.

In addition, the measurement performed by the measuring device is not limited to the measurement using the horseshoe crab hemocyte extract, and may be another measurement.

In addition, in the measuring device 10 of the embodiment, the configuration member may be changed without departing from the spirit of the technology of the present disclosure. For example, the number of the specimen racks 32, the position of the specimen rack 32, or the like can be changed. In addition, the upper cover 14 is slid to the upper side to open the upper opening 12, but the present disclosure is not limited to this configuration. For example, the upper cover 14 may be of a type in which the upper opening is opened by a hinge, similarly to the lower cover 15.

In the measuring device 10 of the embodiment, at least a part of the tray 28 in the pull-out position P6 is included in the imaginary rectangular shape 78 that circumscribes the tip placement area 23 and the dilution plate placement area 30 in plan view, but the technology of the present disclosure is not limited to this configuration. For example, in a case where the tip placement area 23 and the dilution plate placement area 30 have a curved shape other than the rectangular shape, a configuration may be adopted in which at least a part of the tray 28 in the pull-out position P6 is included in an imaginary circular shape that circumscribes the tip placement area 23 and the dilution plate placement area 30 in plan view.

It should be noted that the above contents and illustrations are detailed descriptions of the parts according to the technology of the present disclosure, and are merely examples of the technology of the present disclosure. For example, the description of the configuration, function, action, and effect is description of an example of the configuration, function, action, and effect of the parts according to the technology of the present disclosure. Therefore, it is needless to say that unnecessary parts may be deleted, new elements may be added, or replacements may be made with respect to the above contents and illustrations without departing from the spirit of the technology of the present disclosure. In addition, in order to avoid confusion and to facilitate understanding of the parts according to the technology of the present disclosure, the description of common technical knowledge and the like that do not require specific description in order to enable the implementation of the technology of the present disclosure is omitted in the above contents and illustrations.

### [Preferred Aspects of Present Disclosure]

Hereinafter, preferred aspects of the present disclosure will be described.

### [Supplementary Note 1]

A measuring device comprising:
a measurement part that measures a target substance contained in a sample solution;
a tool placement area on which a tool used for the measurement is placed and that is disposed above the measurement part in a vertical direction;
a dispensing part that dispenses a liquid and is movable above the tool placement area; and
a housing that accommodates the measurement part, the tool placement area, and the dispensing part therein.

### [Supplementary Note 2]

The measuring device according to Supplementary Note 1,
in which the tool includes a tip that is replaceably mounted on a distal end of the dispensing part, and
the tool placement area is a tip placement area on which the tip is placed.

### [Supplementary Note 3]

The measuring device according to Supplementary Note 2,
in which the measurement part includes a tray that is movable in a horizontal direction between a storage position where the tray is stored inside the measurement part and a pull-out position where the tray is pulled out from the storage position to an outside, and on which a measurement plate accommodating the sample solution is replaceably disposed.

### [Supplementary Note 4]

The measuring device according to Supplementary Note 3, further comprising:
a dilution plate placement area on which a dilution plate used for diluting a liquid is placed,
in which the dilution plate placement area is provided below the tip placement area in the vertical direction and above the tray in the vertical direction.

### [Supplementary Note 5]

The measuring device according to Supplementary Note 4,
in which the dilution plate placement area is provided at a position that does not overlap the tray in the pull-out position in plan view.

### [Supplementary Note 6]

The measuring device according to any one of Supplementary Notes 2 to 5, further comprising:
an upper opening and a lower opening that are formed on one surface of the housing and are provided at different positions in the vertical direction;
an upper cover for opening and closing the upper opening; and
a lower cover for opening and closing the lower opening,
in which the upper cover moves between an open position for opening the upper opening and a closed position for closing the upper opening and covering the tip placement area, and
the lower cover moves between an open position for opening the lower opening and a closed position for closing the lower opening.

### [Supplementary Note 7]

The measuring device according to Supplementary Note 6, further comprising:
a specimen rack that is disposed in the housing and stores a specimen container for accommodating a sample stock solution contained in the sample solution,
in which the upper cover covers the specimen rack in the closed position.

### [Supplementary Note 8]

The measuring device according to Supplementary Note 7,
in which the specimen rack is capable of being pulled out to an outside of the housing through the upper opening.

### [Supplementary Note 9]

The measuring device according to any one of Supplementary Notes 6 to 8,
in which the lower cover is configured to cover the measurement part disposed below the tip placement area in the vertical direction and to be opened forward on an upper portion side by a hinge provided on a lower portion side with respect to the housing, and
the upper cover is configured to be opened by moving to an upper side in the vertical direction with respect to the housing.

### [Supplementary Note 10]

The measuring device according to Supplementary Note 1,
in which the measurement part includes a tray that is movable in a horizontal direction between a storage position where the tray is stored inside the measurement part and a pull-out position where the tray is pulled out from the storage position to an outside, and on which a measurement plate accommodating the sample solution is replaceably disposed.

### [Supplementary Note 11]

The measuring device according to any one of Supplementary Notes 1 to 10,
in which the target substance is endotoxin.

The disclosure of Japanese Patent Application No. 2022-023322 is incorporated herein by reference in its entirety.

All documents, patent applications, and technical standards described in the present specification are incorporated in the present specification by reference to the same extent as in a case where the individual documents, patent applications, and technical standards are specifically and individually stated to be incorporated by reference.

## Claims

1. A measuring device comprising:
a measurement part that measures a target substance contained in a sample solution;
a tool placement area on which a tool used for the measurement is placed and that is disposed above the measurement part in a vertical direction;
a dispensing part that dispenses a liquid and is movable above the tool placement area; and
a housing that accommodates the measurement part, the tool placement area, and the dispensing part therein.

2. The measuring device according to claim 1,
wherein the tool includes a tip that is replaceably mounted on a distal end of the dispensing part, and
the tool placement area is a tip placement area on which the tip is placed.

3. The measuring device according to claim 2,
wherein the measurement part includes a tray that is movable in a horizontal direction between a storage position where the tray is stored inside the measurement part and a pull-out position where the tray is pulled out from the storage position to an outside, and on which a measurement plate accommodating the sample solution is replaceably disposed.

4. The measuring device according to claim 3, further comprising:
a dilution plate placement area on which a dilution plate used for diluting a liquid is placed,
wherein the dilution plate placement area is provided below the tip placement area in the vertical direction and above the tray in the vertical direction.

5. The measuring device according to claim 4,
wherein the dilution plate placement area is provided at a position that does not overlap the tray in the pull-out position in plan view.

6. The measuring device according to claim 2, further comprising:
an upper opening and a lower opening that are formed on one surface of the housing and are provided at different positions in the vertical direction;
an upper cover for opening and closing the upper opening; and
a lower cover for opening and closing the lower opening,
wherein the upper cover moves between an open position for opening the upper opening and a closed position for closing the upper opening and covering the tip placement area, and
the lower cover moves between an open position for opening the lower opening and a closed position for closing the lower opening.

7. The measuring device according to claim 6, further comprising:
a specimen rack that is disposed in the housing and stores a specimen container for accommodating a sample stock solution contained in the sample solution,
wherein the upper cover covers the specimen rack in the closed position.

8. The measuring device according to claim 7,
wherein the specimen rack is capable of being pulled out to an outside of the housing through the upper opening.

9. The measuring device according to claim 6,
wherein the lower cover is configured to cover the measurement part disposed below the tip placement area in the vertical direction and to be opened forward on an upper portion side by a hinge provided on a lower portion side with respect to the housing, and
the upper cover is configured to be opened by moving to an upper side in the vertical direction with respect to the housing.

10. The measuring device according to any one of claims 1 to 9,
wherein the target substance is endotoxin.
